# EUROPÄISCHE PATENTSCHRIFT

(11) **EP 1 924 208 B1**
(45) Veröffentlichungstag und Bekanntmachung des Hinweises auf die Patenterteilung: **22.08.2012**
(21) Anmeldenummer: 06793106.3
(22) Anmeldetag: 31.08.2006
(51) Int. Cl.: A61B 17/16, A61F 2/36

(54) **Femur Implantat**
Femur Implant
Implant du femur

(30) Priorität: 31.08.2005 EP 05018940
(43) Veröffentlichungstag der Anmeldung: 28.05.2008
(73) Patentinhaber: Zimmer GmbH, 8404 Winterthur (CH)
(72) Erfinder: HOWALD, Ralph, 9230 Flawil (CH); HEUBERGER, Peter, 8352 Räterschen (CH); HERTIG, Daniel, 8248 Uhwiesen (CH); GREEVE, Job-Jan, 7203 BE Zutphen (NL)
(74) Vertreter: Manitz, Finsterwald & Partner GbR
(86) Internationale Anmeldenummer: PCT/EP2006/065869
(87) Internationale Veröffentlichungsnummer: WO 2007/026003

(56) Entgegenhaltungen:
- EP-A- 0 013 863
- EP-A1- 0 017 930
- FR-A- 2 519 545
- GB-A- 2 139 097
- US-A1- 2003 163 202

## Beschreibung

Die vorliegende Erfindung betrifft ein kappenförmig ausgeformtes Implantat zur Implantation auf einem präparierten Stumpf, wie im Anspruch 1 definiert.

Bei Verwendung eines derartigen kappenförmig ausgeformten Implantats kann der natürliche Femurkopf des Patienten weitgehend erhalten werden. Der Femurkopf wird bei der Operation lediglich an seiner Oberfläche bearbeitet, um das Aufsetzen des kappenförmig ausgeformten Implantats zu ermöglichen. Diese Operationstechnik wird auch als "ReSurfacing" bezeichnet.

Aus EP 1407728 ist ein Resurfacing-Implantat für einen Femurkopf bekanntgeworden, das zur Verankerung mit einem in den Schenkelhals hineinreichenden Zapfen versehen ist. Die US 2003/0163202 offenbart ein Implantat, welches eine konische innere Kontaktfläche zur Befestigung auf einem kegelstumpfförmig präparierten Femurkopf aufweist.

Hier wird nun ein kappenförmig ausgeformtes Implantat der eingangs genannten Art vorgeschlagen, welches, neben einer Vielzahl anderer vorteilhafter Eigenschaften, die Artikulationsfläche des natürlichen Femurkopfes gut nachbildet, mit einem geringen Verlust an Knochenmaterial implantierbar ist, und, sowohl bei zementierter als auch unzementierter Implantation, eine gute Primärverankerung sowie einen sicheren und zuverlässigen Sitz über eine lange Zeit gewährleistet. Spezifischer soll es beispielsweise ermöglicht sein, die Geometrie der natürlichen Artikulationsfläche, welche im Wesentlichen zur Halsachse des Femurkopfes symmetrisch ist, durch eine von der Außenfläche der Femurkappe gebildete künstliche Artikulationsfläche nachzubilden und gleichzeitig eine sichere Befestigung des Implantats auf dem Femurkopf zu gewährleisten. Dabei ist festzuhalten, dass die Hauptangriffsrichtung der in den Femurkopf eingeleiteten Kraft näherungsweise einer vom Femurkopf zum distalen Ende des Femur gerichteten Linie folgt oder gar noch stärker von der Achse des Femurhalses abweicht. Gemäß einem weiteren Aspekt soll das Implantat derart angegeben werden, dass es sicher auf dem präparierten Stumpf eines Femurkopfes verankerbar ist. Weiterhin soll ein Verfahren zur Implantation eines kappenförmig ausgeformten Implantats angegeben werden. Es soll auch ein Verfahren zur Herstellung des beschriebenen kappenförmig ausgeformten Implantats angegeben werden. Weiterhin soll ein Instrument zur Herstellung eines kappenförmig ausgeformten Implantats angegeben werden.

Neben anderen vorteilhaften Eigenschaften vermögen die in den Patentansprüchen beschriebenen Gegenstände auch diese Forderungen zu erfüllen. Eigenschaften, wie beispielsweise die einfache Handhabbarkeit durch den Chirurgen, die Implantierbarkeit mit. minimalen Inzisionen, oder ein minimaler Knochengewebsverlust und weichteilschonende Implantation, werden als selbstverständlich gestellt und als zu erfüllen betrachtet.

Das angegebene Implantat weist die Form eines Kugelabschnitts auf, mit einer ebenen Basisfläche und einer senkrecht zu der Basisfläche angeordneten Polachse. In dem Kugelabschnitt ist ein rotationssymmetrischer Hohlraum angeordnet, der von der Basisfläche ausgeht, das heisst eine Öffnung aufweist, die wenigstens zum Teil in der Basisfläche liegt. Der Hohlraum weist eine Hohlraumachse auf, die unter einem von Null verschiedenen Winkel zur Polachse angeordnet ist. Dies ermöglicht es, dass das Implantat derart auf den präparierten Stumpf aufgesetzt werden kann, dass die Polachse des Implantats mit der Halsachse des Femurkopfes zusammenfällt. Gleichzeitig kann die Befestigung des Implantats derart vorgenommen werden, dass die Hohlraumachse des Hohlraums des Implantats wenigstens näherungsweise mit der Hauptbelastungsrichtung des Femurkopfes zusammenfällt.

Die Hohlraumachse schneidet in einer Ausführungsform die Polachse, insbesondere in einem Kugelmittelpunkt des Kugelabschnitts. Die Polund Hohlraumachse schließen miteinander einen Winkel im Bereich von 15° bis 50°, insbesondere 15° bis 35°, ein. Dies deckt zum Beispiel den Bereich ab, in dem der Neigungswinkel der Hauptbelastungsrichtung gegen die Halsachse geneigt ist. Der Kugelabschnitt weist beispielsweise einen Kugeldurchmesser im Bereich von 38 mm bis 60 mm und eine in Richtung der Polachse von der Basisfläche bis zum Pol messende Höhe von 60% bis 80% des Kugeldurchmessers auf. Der genannte Durchmesserbereich ist typisch für Prothesen, bei denen der Schenkelhals erhalten wird, um die Beweglichkeit zu gewährleisten, und wird beispielsweise im Bereich des Durchmessers des natürlichen Femurkopfes gewählt. Bei diesen Durchmessern finden beispielsweise Femurkomponenten aus Metall Verwendung, wie sie auch in der EP 892627 beschrieben worden sind, und beispielsweise auch in Kombination mit einer metallischen Gegenlauffläche, was ebenfalls aus der EP 892627 bekanntgeworden ist; wobei diese Schriften einen integrierenden Bestandteil der vorliegenden Beschreibung darstellen. Die über die Polachse gemessene Kappenhöhe gewährleistet einen hinreichend grossen Winkelbereich, in dem die Femurkappe eine wirksame Artikulationsfläche zur Verfügung stellt.

Der Hohlraum weist eine Innenfläche auf, welche eine Durchdringung mit der Oberfläche des Kugelabschnitts aufweist, wobei wenigstens ein Umfangssegment der Innenfläche eine Durchdringung mit einem sphärischen Oberflächenbereich des Kugelabschnitts aufweist. Insbesondere können ein erstes Umfangssegment der Innenfläche den sphärischen Oberflächenbereich des Kugelabschnitts und ein zweites Umfangssegment der Innenfläche die Basisfläche durchdringen. Das Implantat kann also eine Kappenöffnung aufweisen, welche durch einen Rand des Implantats begrenzt ist, welcher Rand nur bereichsweise, d.h. nicht vollumfänglich, in der Ebene der Basisfläche angeordnet ist. Ein erster Bereich des Randes kann in der Ebene der Basisfläche und ein zweiter Bereich des Randes kann in einer Eintrittsebene, die senkrecht zur Hohlraumachse orientiert ist, angeordnet sein. Das heisst, die Öffnung des Implantats ist dann nicht eben, sondern in zwei Teilflächen aufgeteilt, die jeweils senkrecht zur Polachse und senkrecht zur Hohlraumachse angeordnet sind.

In einer weiteren Ausführungsform weist der Hohlraum die Form eines Kegelstumpfes auf, welcher sich entlang der Hohlraumachse in einer Richtung verjüngt, die ins Innere des Implantats hineinweist. Durch die Kegelstumpfform wird die Stimulierung des Knochengewebes am präparierten Stumpf erhöht, um einem Abbau von Knochenmaterial entgegenzuwirken respektive das Anwachsen von Knochenmaterial an das Implantat zu stimulieren, indem der Konus derart ausgelegt wird, dass in allen Bereichen der Grenzfläche zwischen Stumpf und Implantat-Innenfläche Druckbelastung vorliegt. Ferner sind konische Flächen einfach herstellbar. Der Kegelstumpf weist einen vollen Konusöffnungswinkel von beispielsweise 10° bis 65°, insbesondere 16° bis 50°, auf.

In einer Ausführungsform des Implantats sind im Inneren des Hohlraums Verankerungsmittel zur Verankerung des Implantats auf dem Stumpf angeordnet, welche an der Innenfläche des Implantats, welche den Hohlraum begrenzt, angeordnet sind. Diese dienen bedarfsweise einer weiter verbesserten Primärverankerung des Implantats auf dem Knochen und sind auch als Sicherung gegen das Verdrehen des Implantats auf dem Knochenstumpf verwendbar, wohingegen eine Ausführungsform ohne Verankerungsmittel eine geringer invasive Implantation mit einem geringeren Verlust an Knochenmaterial ermöglicht. Generell ist das beschriebene Implantat zur zementfreien Implantation vorgesehen, wobei aber auch eine Implantation unter Verwendung von Knochenzement selbstverständlich prinzipiell möglich ist. Die Verankerungsmittel sind in einer Ausführungsform symmetrisch um die Hohlraumachse verteilt angeordnet. Bei einer Anordnung der Verankerungsmittel am Rand des Hohlraums bleibt das Knochengewebe in der Mitte des präparierten Stumpfes des Femurkopfes unversehrt. In einer beispielhaften Ausführungsform des Implantats umfassen die Verankerungsmittel am Umfang des Hohlraums angeordnete widerhakenartige Strukturen, welche von einer Öffnung des Hohlraums wegweisend ins Innere des Implantats hinein gebogen sind. Die widerhakenartigen Strukturen sind beispielsweise von einer Vielzahl von Verankerungszähnen gebildet, deren Höhe im Zehntelmillimeter- bis Millimeterbereich liegt, zum Beispiel um 1 mm, beispielsweise im Bereich von 0.5 mm bis 1.5 mm. Beispielsweise sind diese Strukturen konzentrisch um die Hohlraumachse angeordnet. In einer weiteren Ausbildung des Implantats umfassen die Verankerungsmittel mehrere Verankerungsrippen, -finnen und/oder parallel zur Hohlraumachse orientierte Verankerungsstifte, die insbesondere der Primärverankerung dienen. Bei Verankerungsfinnen können den Finnen entsprechende Ausnehmungen im Femurkopf auf einfache Weise durch Einsägen oder Einschneiden des Femurkopfes mit einem entsprechenden Sägeblatt hergestellt werden. Hinsichtlich der Anzahl der einzelnen Verankerungselemente kann zwischen einem Verlust an Knochenmaterial einerseits und bestmöglicher Verankerung andererseits abgewogen werden.

Um ein Einwachsen von Knochenmaterial in das Implantat zu fördern, kann der Hohlraum von einer Innenfläche begrenzt sein, die zumindest bereichsweise rauhgestrahlt ist und/oder eine Mikrostruktur aufweist. Beispielsweise weist eine Innenfläche des Implantats, welche den Hohlraum umgibt, eine Titan-Plasma-Beschichtung auf. Die Innenfläche kann auch mit einer porösen Struktur versehen sein, deren Oberfläche mit Metall, insbesondere Tantal, umgeben ist. Eine solche Oberfläche ist beispielsweise unter dem Handelsnamen "Trabecular Metal" des Implantateherstellers ZIMMER bekanntgeworden.

Die verschiedenen oben angegebenen Ausführungsformen eines Implantats gemäss dem Anspruch 1 beziehungsweise die dort realisierten Merkmale können selbstverständlich untereinander kombiniert werden.

Bei einem möglichen Verfahren zur Implantation eines Implantats wird ein kappenförmig ausgeformtes Implantat gewählt, welches einen Hohlraum mit einer Hohlraumachse umfasst. Der Femurkopf wird derart reseziert, um einen präparierten Stumpf zu erhalten, der eine Gegenform zu dem Hohlraum des Implantats darstellt und rotationssymmetrisch bezüglich einer Implantationsachse ist, und zwar derart, dass die Implantationsachse wenigstens in einer Frontalebene, allenfalls auch, wie unten beschrieben, zusätzlich in einer Sagittalebene gegenüber der Femurhalsachse geneigt ist. Die Neigung in der Frontalebene liegt beispielsweise im Bereich von 15° bis 50°, insbesondere 15° bis 35°, und wird allgemein derart bemessen sein, dass die Implantationsachse mit der Richtung der Hauptbelastung des Femurkopfes übereinstimmt. Insbesondere kann ein Implantat ausgewählt werden, welches ein Implantat der oben beschriebenen Art ist.

In einer Ausführungsform des Verfahrens zur Implantation eines Implantats werden vor dem Resezieren des Femurkopfes ein erster Bohrdraht in den Femurkopf entlang der Femurhalsachse eingesetzt, ein Ausrichtinstrument auf den ersten Bohrdraht aufgesetzt, ein zweiter Bohrdraht in den Femurkopf entlang der gegenüber der Femurhalsachse geneigten Implantationsachse eingesetzt, und das Ausrichtinstrument und der erste Bohrdraht entfernt, wobei die vorstehend genannten Schritte insbesondere in dieser Reihenfolge ausgeführt werden. Instrumente und Verfahren, mit deren Hilfe der erste Bohrdraht auf dem Femurkopf zentriert und/oder mit der Halsachse in Übereinstimmung gebracht werden kann, sind bekannt, so beispielsweise das Zentrierinstrument für die Implantation der DUROM Hüftkappe von ZIMMER. Das Ausrichtinstrument umfasst ein Anzeige- oder Lehrmittel, welches dazu dient, die Neigung der Implantationsachse zur Halsachse festzulegen und damit die Implantationsachse festzulegen.

Mit geeigneten Hilfsmitteln und/oder Verfahren kann alternativ der zweite Bohrdraht auch unmittelbar, ohne Zuhilfenahme eines ersten Bohrdrahtes, implantiert werden. Der zweite Bohrdraht wird beispielsweise als Führungswerkzeug bei der Bearbeitung des Femurkopfes benutzt und/oder vor dem Aufsetzen des kappenförmig ausgeformten Implantats auf den präparierten Stumpf entfernt.

In einer Ausführungsform umfasst das Verfahren zur Implantation eines Implantats, insbesondere eines Implantats der oben beschriebenen Art, das Implantat derart zu implantieren, dass beim Aufsetzen des kappenförmig ausgeformten Implantats auf den präparierten Stumpf die Hohlraumachse und die Implantationsachse zusammenfallen. Das Verfahren zur Implantation eines Implantats kann zusätzlich vorsehen, das Implantat gegen Verkippung der Hohlraumachse gegenüber der Implantationsachse zu sichern. Die Sicherung kann in einer Führung für das Implantat, welche Führung in Richtung der Implantationsachse ausgerichtet ist, bestehen. Eine Ausführungsform umfasst, den Femurkopf derart zu bearbeiten, dass die Neigung der Implantationsachse gegenüber der Femurhalsachse dem Winkel zwischen der Polachse und der Hohlraumachse des Implantats entspricht. Weiterhin wird in einer Ausführungsform das Implantat derart aufgesetzt, dass die Polachse des Implantats und die Femurhalsachse übereinstimmen. Eine weitere beispielhafte Ausführungsform des Verfahren zur Implantation eines Implantats umfasst, den Femurkopf derart zu bearbeiten, dass die Implantationsachse sowohl in der Frontalebene als auch in einer Sagittalebene gegenüber der Femurhalsachse geneigt ist, also eine sogenannte Antetorsion aufweist.

Die verschiedenen oben angegebenen Ausführungsformen eines Implantationsverfahrens können selbstverständlich untereinander kombiniert werden.

Bei einen möglichen Verfahren zur Herstellung eines kappenförmig ausgeformten Implantats für einen präparierten Stumpf eines Femurkopfes, insbesondere des vorstehend angegebenen Implantats, werden ein Kugelabschnitt mit einer ebenen Basisfläche und einer senkrecht zu der Basisfläche angeordneten Polachse hergestellt, und ein rotationssymmetrischer Hohlraum mit einer Hohlraumachse in dem Vollkugelabschnitt ausgehend von der Basisfläche derart erzeugt, dass die Hohlraumachse unter einem von Null verschiedenen Winkel zur Polachse angeordnet ist. Dabei kann beispielsweise erst der Kugelabschnitt mit der ebenen Basisfläche hergestellt und danach der Hohlraum eingebracht werden, oder es wird zunächst in einer Kugel oder einem Kugelabschnitt der Hohlraum hergestellt, und durch ein geeignetes Verfahren eine plane Fläche hergestellt, welche senkrecht zu einer Polachse angeordnet ist, welche mit der Hohlraumachse einen von Null verschiedenen Winkel einschliesst.

Bei einem weiteren Verfahren zur Herstellung eines kappenförmig ausgeformten Implantats für einen präparierten Stumpf eines Femurkopfes, insbesondere des vorstehend angegebenen Implantats, werden an der Innenfläche des Hohlraums Rillen erzeugt, beispielsweise mittels eines Drehverfahrens, um an der Innenfläche Erhebungen in Form von konzentrischen Ringen zu erhalten, welche konzentrisch um die Hohlraumachse angeordnet sind. Schließlich werden die ringförmigen Erhebungen mittels eines Prägeverfahrens eingekerbt, um aus den konzentrischen Ringen jeweils mehrere Verankerungszähne zu erzeugen. Dabei wird der Hohlraum bei seiner Herstellung gegenüber dem angestrebten Hohlraumendmass mit einem Durchmesser-Untermass erzeugt. In einer Ausführungsform dieses Verfahrens werden die Kerben unter Anwendung einer Kraft erzeugt, die in Richtung der Hohlraumachse in den Hohlraum hineinweist. Dadurch werden die verbleibenden Erhebungen in Eckenbereichen, die benachbart zu den Kerben angeordnet sind, plastisch derart verformt, dass sie in den Hohlraum hineinweisen. Dadurch wird eine Widerhakenstruktur zum Verankern des Implantats geschaffen. Insbesondere kann ein Instrument zum Erzeugen der Einkerbungen, insbesondere ein Prägeinstrument, in Richtung der Hohlraumachse in den Hohlraum eingeführt und unter Anwendung einer in Richtung der Hohlraumachse in den Hohlraum hineingerichteten Kraft gegen die Erhebungen gedrückt werden, um die Einkerbungen zu erzeugen. Ein Instrument zur Durchführung eines solchen Verfahrens umfasst ein Schaftteil und ein Kopfteil. Mit dem Schaftteil kann das Instrument gegriffen werden. Das mit dem Schaftteil verbundene Kopfteil weist wenigstens zwei Prägekanten zum Erzeugen der Einkerbungen auf. Die Prägekanten weisen in einer Seitenansicht Konturen auf, welche eine Gegenform zu der Innenfläche des Hohlraums in einem zur Hohlraumachse parallelen Schnitt des Implantats sind.

Das beschriebene Verfahren zur Herstellung von Verankerungselementen im Inneren von Implantaten ist selbstverständlich nicht auf das kappenförmige Element beschränkt, sondern kann bei anderen Implantaten Anwendung finden, die einen zum Kontakt mit Knochen vorgesehenen Innenraum aufweisen, an dem eine Primärverankerung und ein Anwachsen stattfinden soll.

Die Bearbeitung der sphärischen Artikulationsfläche des Implantats erfolgt beispielsweise mit einem in EP 1 340 477 beschriebenen Verfahren.

Ein Verfahren zur Implantation eines Implantats umfasst, ein kappenförmig ausgeformtes Implantat zu wählen, welches einen Hohlraum mit einer Hohlraumachse umfasst, den Femurkopf derart zu resezieren, um einen präparierten Stumpf zu erhalten, der eine Gegenform zu dem Hohlraum des Implantats darstellt und rotationssymmetrisch bezüglich einer Implantationsachse ist, und den Femurkopf derart zu bearbeiten, dass die Implantationsachse wenigstens in einer Frontalebene gegenüber der Femurhalsachse geneigt ist.

Ein weiteres Verfahren umfasst weiter, vor dem Resezieren des Femurkopfes, einen ersten Bohrdraht in den Femurkopf entlang der Femurhalsachse einzusetzen, ein Ausrichtinstrument auf den ersten Bohrdraht aufzusetzen, welches Ausrichtinstrument ein Anzeige- oder Lehrmittel umfasst, welches dazu dient, die Neigung der Implantationsachse zur Halsachse festzulegen und damit die Implantationsachse festzulegen, einen zweiten Bohrdraht in den Femurkopf entlang der gegenüber der Femurhalsachse geneigten Implantationsachse einzusetzen, und das Ausrichtinstrument und den ersten Bohrdraht zu entfernen.

Dabei findet in einer Ausführungsform des Verfahrens auch ein geeignetes, zum Beispiel mechanisches, Hilfsmittel Verwendung, um den Mittelpunkt des Femurkopfes zu lokalisieren, um das Ausrichtinstrument so anzusetzen, dass das Zentrum der Drehung zwischen Halsachse und Implantationsachse wenigstens im Wesentlichen mit dem Mittelpunkt des Femurkopfes übereinstimmt.

Ein weiteres Verfahren umfasst weiter, den zweiten Bohrdraht als Führungswerkzeug bei der Bearbeitung des Femurkopfes zu benutzen.

Ein weiteres Verfahren umfasst weiter, vor dem Aufsetzen des kappenförmig ausgeformten Implantats auf den präparierten Stumpf, den zweiten Bohrdraht zu entfernen.

Ein weiteres Verfahren umfasst weiter, das Implantat derart zu implantieren, dass beim Aufsetzen des kappenförmig ausgeformten Implantats auf den präparierten Stumpf die Hohlraumachse und die Implantationsachse zusammenfallen.

Ein weiteres Verfahren umfasst weiter, das Implantat gegen Verkippung der Hohlraumachse gegenüber der Implantationsachse zu sichern.

Ein weiteres Verfahren umfasst weiter, ein Implantat auszuwählen, das ein Implantat gemäß zumindest einem der Ansprüche 1 bis 15 ist.

Ein weiteres Verfahren umfasst weiter, den Femurkopf derart zu bearbeiten, dass die Neigung der Implantationsachse gegenüber der Femurhalsachse dem Winkel zwischen der Polachse und der Hohlraumachse des Implantats entspricht.

Ein weiteres Verfahren umfasst weiter, das Implantat derart aufzusetzen, dass die Polachse des Implantats und die Femurhalsachse übereinstimmen.

Ein weiteres Verfahren umfasst weiter, den Femurkopf derart zu bearbeiten, dass die Implantationsachse sowohl in der Frontalebene als auch in einer Sagittalebene gegenüber der Femurhalsachse geneigt ist.

Nachfolgend wird die Erfindung anhand von in der Zeichnung illustrierten Ausführungsbeispielen näher erläutert. Dabei sollen die Ausführungsbeispiele und die Zeichnung nur instruktiv verstanden werden und sollen nicht zur Einschränkung der in den Ansprüchen beschriebenen Gegenstände dienen. Die Darstellungen in der Zeichnung sind vereinfacht; für das Verständnis der Erfindung nicht notwendige Einzelheiten sind weggelassen worden.
- Fig. 1: zeigt zwei Ausführungsformen eines Implantats.
- Fig. 2: zeigt weitere Ausführungsformen eines Implantats.
- Fig. 3: zeigt den proximalen Teil eines Femurs mit einem aufgesetzten Implantat.
- Fig. 4: zeigt eine weitere Ausführungsform eines Implantats mit stiftartigen Verankerungselementen.
- Fig. 5: zeigt eine andere Ausführungsform eines Implantats mit finnenartigen Verankerungselementen.
- Fig. 6: zeigt eine Ausführungsform eines Prägeinstruments zur Herstellung von Verzahnungen im Hohlraum.
- Fig. 7: zeigt eine Ausführungsform eines Ausrichtinstruments zur Festlegung der Neigung einer Implantationsachse zu einer Femurhalsachse.

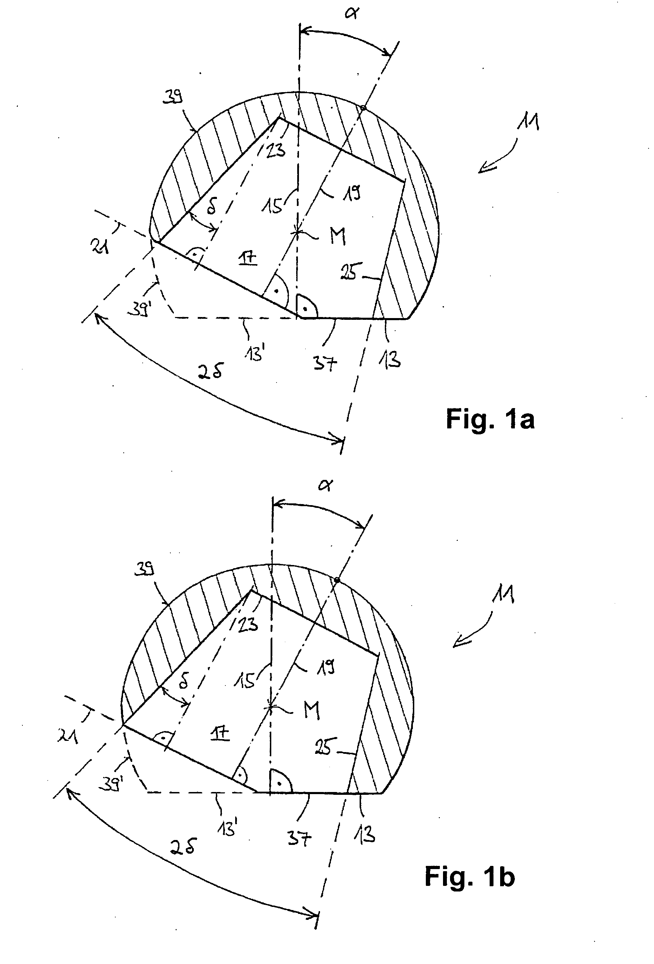

Die Fig. 1a und 1b zeigen jeweils eine Ausführungsform eines kappenförmig ausgeformten Implantats zur Implantation auf einem in den Fig. 1a und 1b nicht dargestellten präparierten Stumpf. Das Implantat weist die Form eines Kugelabschnitts 11 mit einem Kugelmittelpunkt M, einem sphärischen Oberflächenbereich 39 und einer ebenen Basisfläche 13 auf, wobei senkrecht zur Basisfläche 13 eine Polachse 15 angeordnet ist. Daneben weist der Kugelabschnitt 11 einen Hohlraum 17 auf, der rotationssymmetrisch um eine Hohlraumachse 19 ausgebildet ist. In gestrichelter Darstellung ist ein Kugelabschnitt mit einem sphärischen Oberflächenbereich 39' und einer Basisfläche 13' angedeutet, welcher Kugelabschnitt keinen Hohlraum aufweist, d.h. welcher Kugelabschnitt nicht mit einem Hohlraum überlagert ist. Der Hohlraum 17 weist die Form eines Kegelstumpfes auf, welcher sich entlang der Hohlraumachse 19 in einer ins Innere des Implantats weisenden Richtung verjüngt. Der Hohlraum 17 ist von einer Innenfläche 23, 25 begrenzt, die eine Stirnfläche 23 und eine konische Mantelfläche 25 mit einem Konusöffnungswinkel 2δ bzw. einem halben Konusöffnungswinkel δ umfasst. Die Hohlraumachse 19 und die Polachse 15 schließen einen von Null verschiedenen Winkel α ein und schneiden sich im Mittelpunkt M des Kugelabschnitts 11. Die Mantelfläche 25 des Hohlraums 17 des in Fig. 1 gezeigten Implantats durchdringt dabei sowohl einen Teil des sphärischen Oberflächenbereichs 39 als auch einen Teil der ebenen Basisfläche 13. Der Hohlraum 17 weist somit eine Kappenöffnung auf, welche durch einen Rand 37 begrenzt ist, wobei wenigstens ein erster Teil des Randes 37 durch die Durchdringung der Mantelfläche 25 mit dem sphärischen Oberflächenbereich 39 gebildet ist und in einer Eintrittsebene 21 angeordnet ist, die zu der Hohlraumachse 19 senkrecht angeordnet ist. In dem dargestellten Beispiel liegt ein zweiter Teil des Randes in der Basisfläche 13. In der Ausführungsform gemäß Fig. 1b ist der erste Teil des Randes als Kante ausgebildet, welche sich aus der Überlagerung des gestrichelt dargestellten Kugelabschnitts und des Hohlraums 17 ergibt. In der Ausführungsform gemäß Fig. 1a ist der erste Teil des Randes verrundet. Grundsätzlich kann der Hohlraum auch eine von einem Kegelstumpf abweichende rotationssymmetrische Form, beispielsweise die Form eines Sphäroids oder Ellipsoids oder eines Zylinders, aufweisen. Das in den Fig. 1a und 1b gezeigte Implantat kann beispielsweise dadurch hergestellt werden, dass in einem Vollkugelabschnitt mit einem sphärischen Oberflächenbereich, einer ebenen Basisfläche und einer senkrecht auf der ebenen Basisfläche stehenden Polachse ausgehend von der ebenen Basisfläche ein um eine Hohlraumachse rotationssymmetrischer Hohlraum unter einem Winkel gegenüber der Polachse erzeugt wird, oder in einer Vollkugel oder in einem Vollkugelabschnitt der Hohlraum mit der Hohlraumachse erzeugt wird, und danach eine plane Basisfläche senkrecht zur Polachse hergestellt wird.

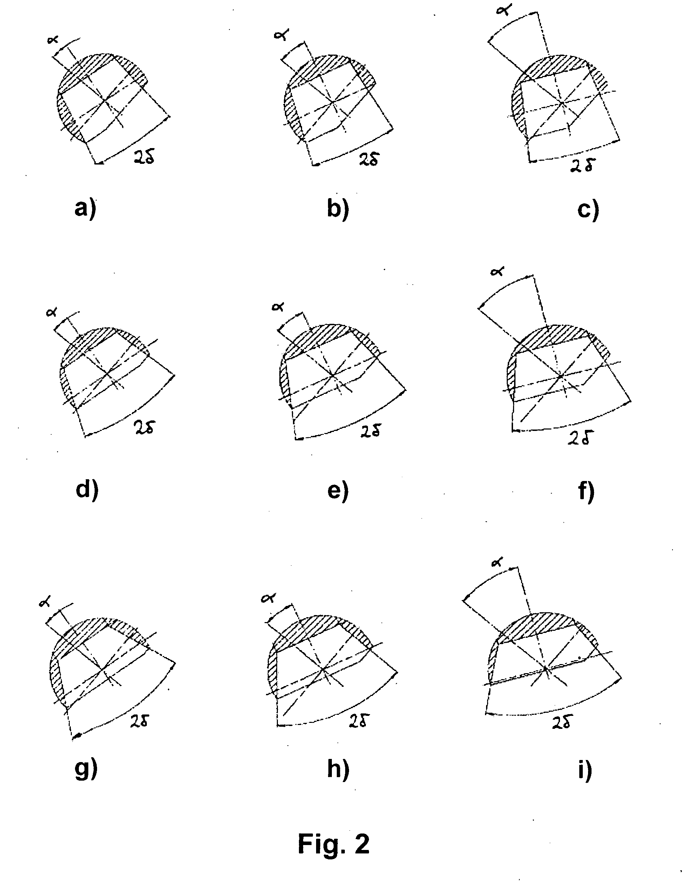

Die in den Fig. 2a bis 2i gezeigten Ausführungsformen unterscheiden sich durch ihre Konusöffnungswinkel 2δ und/oder durch die jeweils zwischen der Hohlraumachse 19 und der Polachse 15 ausgebildeten Winkel α voneinander, um verschiedene, in der Praxis vorkommende Varus-/ Valgusstellungen und/oder CCD-Winkel abdecken zu können. Die Ausführungsformen gemäß den Fig. 2a bis 2c weisen jeweils einen Konusöffnungswinkel 2δ von 16° auf. Die Ausführungsformen gemäß den Fig. 2d bis 2f weisen jeweils einen Konusöffnungswinkel 2δ von 35° auf. Die Ausführungsformen gemäß den Fig. 2g bis 2i weisen jeweils einen Konusöffnungswinkel 2δ von 50° auf. Die Ausführungsformen gemäß den Fig. 2a, 2d und 2g weisen jeweils einen Winkel α von 15° auf. Die Ausführungsformen gemäß den Fig. 2b, 2e und 2h weisen jeweils einen Winkel α von 25° auf. Die Ausführungsformen gemäß den Fig. 2c, 2f und 2i weisen jeweils einen Winkel α von 35° auf.

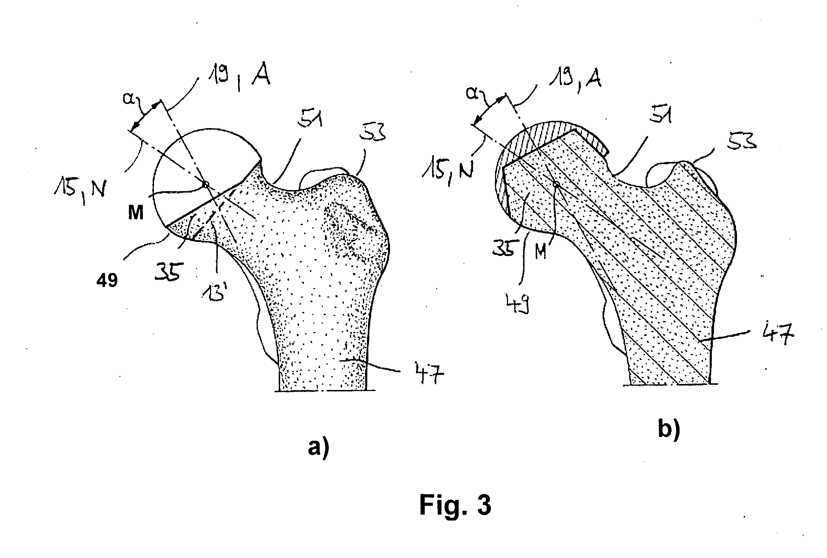

Fig. 3a zeigt eine Seitenansicht eines Femurs 47 mit einem Femurkopf 49, einem Femurhals 51 und einem großen Trochanter 53, wobei auf dem Femurkopf 49 ein Implantat implantiert ist. Ferner ist die ebene Basisfläche 13' des in Fig. 1 angedeuteten Kugelabschnitts ebenfalls gestrichelt dargestellt. Fig. 3b zeigt einen Längsschnitt durch das Femur 47 und das Implantat aus Fig. 3a. Die äußere Form des nach der Präparation verbleibenden Femurstumpfes 35 gemäß Fig. 3b entspricht der durch den Hohlraum 17 bzw. die Innenfläche 23, 25 gebildeten Innenform des Implantats. Der Hohlraum 17 und der präparierte Femurstumpf 35 bilden Gegenformen zueinander. Hierdurch sitzt das implantierte Implantat vollflächig auf dem präparierten Femurstumpf 35 auf, wobei insbesondere eine kegelstumpfförmige Kontaktfläche zwischen Femurstumpf 35 und Implantat einen Kegelsitz als Primärverankerung ermöglicht und bei entsprechender Wahl des Kegelwinkels eine permanente Druckbelastung auf den Knochen wirkt, so dass eine permanente Knochenstimulation erreicht wird. Hierdurch wird einem Knochenabbau aufgrund fehlender mechanischer Stimulation entgegengewirkt.

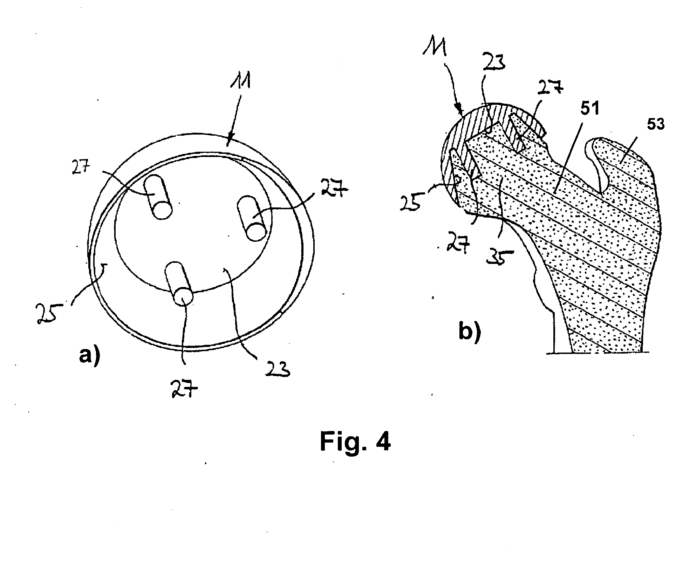

Um die Halterung des Implantats auf dem präparierten Stumpf 35 zu verstärken, ist das Implantat gemäß einer weiteren in Figur 4a dargestellten Ausführungsform mit mehreren, parallel zur Hohlraumachse orientierten Verankerungsstiften 27 versehen, die an der Stirnseite 23 der Innenfläche 23, 25 des Implantats angebracht sind und in den Hohlraum 17 vorstehen. Die Verankerungsstifte 27 sind symmetrisch um die Hohlraumachse verteilt. Die Verankerung des Implantats nach dieser Ausführungsform ist in Fig. 4b gezeigt.

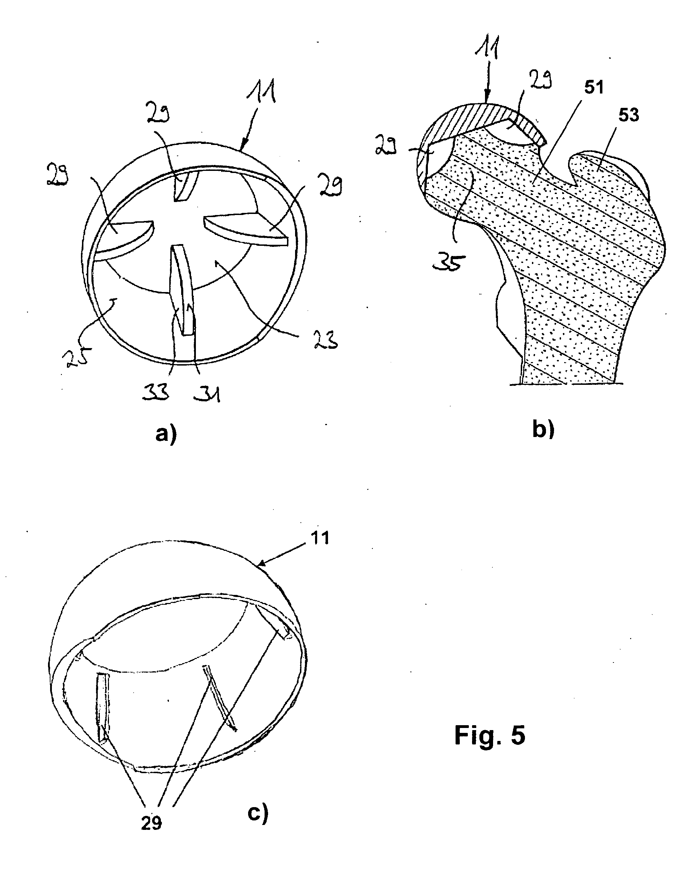

In einer anderen Ausführungsform des Implantats in Fig. 5a sind als Verankerungselemente Verankerungsfinnen 29 vorgesehen, deren Flachseiten 33 parallel zur Hohlraumachse verlaufen und die ebenfalls symmetrisch um die Hohlraumachse verteilt sind. Die Verankerungsfinnen 29 grenzen sowohl an die Stirnfläche 23 als auch an die konische Mantelfläche 25 der Innenseite 23, 25 des Implantats an. Die nach innen weisenden Randseiten 31 der Finnen 29 liegen jeweils auf einem Kreis um einen außerhalb der Außenfläche 13, 39 des Implantats befindlichen Mittelpunkt. Die Verankerung des Implantats nach dieser Ausführungsform ist in Fig. 5b gezeigt. Die zur Aufnahme der Verankerungsfinnen 29 im präparierten Femurkopf 35 erforderlichen Schlitze können einfach durch Einschneiden oder Einsägen mit einem Schneidinstrument hergestellt werden, welches beispielsweise mittels einer geeigneten Lehre geführt ist. In der Figur 5c ist eine weitere beispielhafte Ausführungsform des Implantats mit im Hohlraum angeordneten Finnen 29 gezeigt.

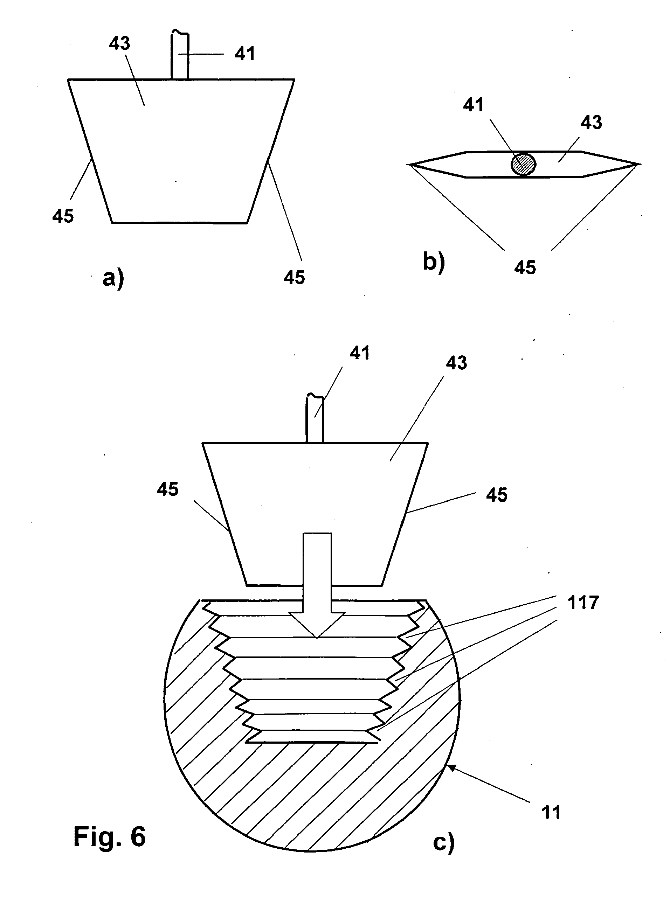

Die Stirnfläche oder die Mantelfläche oder die gesamte Innenfläche eines Implantats der beschriebenen Art ist beispielsweise rauhgestrahlt. Dadurch wird das Einwachsen von Knochenmaterial in das Implantat gefördert. Zur besseren Halterung des Implantats kann die konische Mantelfläche ferner widerhakenartige Strukturen aufweisen, wie sie beispielsweise aus der EP 0639356, welche Schrift insofern einen integrierenden Bestandteil dieser Beschreibung darstellt, für die Verwendung an der Aussenfläche einer Hüftschale bekanntgeworden sind, beispielsweise eine Vielzahl von Verankerungszähnen, die konzentrisch um die Hohlraumachse angeordnet und von der Öffnung des Hohlraums wegweisend ins Innere des Implantats hinein gebogen sind. Die widerhakenartige Struktur kann beispielsweise dadurch hergestellt werden, dass an der Innenfläche eines um eine Hohlraumachse symmetrischen Hohlraums 17 eines Kugelabschnitts 11 zunächst durch Drehen Umfangsrillen erzeugt werden, so dass zwischen den Rillen Erhebungen 171 in Form von konzentrischen Ringen um die Hohlraumachse entstehen; dies ist in der Figur 6c schematisch dargestellt. Danach wird das Instrument 43, das in Fig. 6a in einer Seitenansicht und in Fig. 6b in einer Draufsicht dargestellt ist, in den Hohlraum eingeführt und unter Anwendung einer in Richtung der Hohlraumachse in den Hohlraum hineingerichteten Kraft gegen die Erhebungen gedrückt, wie durch den Pfeil in Figur 6c angedeutet, um dadurch die ringförmigen Erhebungen mittels eines Prägeverfahrens einzukerben, so dass aus jeder ringförmigen Erhebung eine Vielzahl von Verankerungszähnen entsteht. Dabei werden die Verankerungszähne zumindest in Eckbereichen zusätzlich plastisch verformt und ins Innere des Implantats hineinweisend gebogen. Bei diesem Verfahren ist zu beachten, dass der ursprünglich erzeugte Hohlraum mit einem Durchmesser-Untermass gegenüber dem angestrebten Mass, nach dem sich die Präparation des Stumpfes bemisst, hergestellt werden muss, da der Hohlraum in seinem Durchmesser durch die einzuarbeitenden Rillen ja noch vergrössert wird. Die in Fig. 6 gezeigte Ausführungsform eines Instruments zum Erzeugen der vorstehend erläuterten Einkerbungen weist einen Schaft 41 zum Greifen des Instruments und ein mit dem Schaft 41 verbundenes Kopfteil 43 auf. Das Kopfteil 43 umfasst zwei bezüglich des Schafts 41 gegenüberliegende Prägekanten 45, die in der Seitenansicht von Fig. 6a oder 6c Konturen aufweisen, welche eine Gegenform zu der Mantelfläche des Hohlraums 17 sind. Das Instrument kann auch sternartig mit mehreren am Umfang verteilten Prägekanten ausgeführt sein, wodurch entlang des Umfangs des Hohlraums eine Vielzahl von Zähnen in einem Arbeitsgang hergestellt werden kann.

Das Implantat liegt beispielsweise in Form eines Implantatsatzes mit unterschiedlichen Durchmessern der sphärischen Artikulationsfläche und/oder unterschiedlichen Winkeln zwischen Polachse und Hohlraumachse und/oder unterschiedlichen Kegelwinkeln vor, mit dem alle in der Praxis vorkommenden Femurkopfgrößen, CCD-Winkel und weitere Indikationen abgedeckt werden können. Die Kugeldurchmesser der Kugelabschnitte erstrecken sich beispielsweise über den Bereich von 38 mm bis 60 mm.

Der zur Herstellung des Implantats verwendete Werkstoff ist nicht primär erfindungswesentlich; bei den hier betrachteten Durchmessern der Artikulationsfläche werden aber in der Praxis aus Gründen der Tribologie harte, abriebfeste Werkstoffe, wie, nach dem Stand der Technik Metall oder Keramik, Verwendung finden. Das Implantat findet dann beispielsweise in Verbindung mit einer geeigneten Hüftpfanne mit einer Artikulationsfläche aus entsprechend abriebfestem Werkstoff Verwendung, so zum Beispiel als metallische Femurkomponente mit einer acetabularen Komponente mit einer Artikulationsfläche aus Metall oder hochvernetztem Polyethylen oder als keramische Femurkomponente mit einer acetabularen Komponente mit einer Artikulationsfläche aus Keramik oder hochvernetztem Polyethylen. In jüngerer Zeit hat es aber auch schon Bestrebungen gegeben, die Artikulationsflächen von Resurfacingimplantaten aus einem weichen, relativ nachgiebigen Material zu auszuführen, welche dazu vorgesehen sind, gegen eine acetabulare Artikulationsfläche mit ähnlichen Härte- , beziehungsweise "Weichheits-", -eigenschaften zu artikulieren..

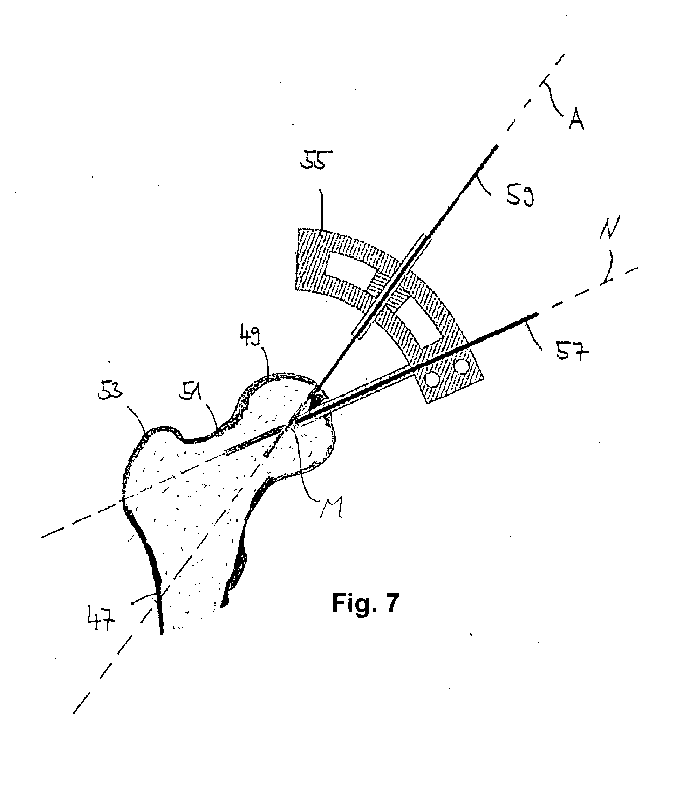

Bei einem beispielhaften Verfahren zur Implantation eines oben erläuterten kappenförmig ausgeformten Implantats auf einem Femurkopf wird gemäß Fig. 7 zunächst ein erster Bohrdraht 57 in den Femurkopf 49 entlang der Femurhalsachse N eingesetzt. Anschließend wird eine Implantationsachse A für das Implantat festgelegt, die zumindest im Wesentlichen der Richtung der Hauptbelastung auf den Femurkopf 49 entspricht. Dazu wird ein Ausrichtinstrument 55 mit einem Anzeigeund/oder Lehrmittel auf den ersten Bohrdraht 57 aufgesetzt, um die Neigung der Implantationsachse A zur Halsachse N festzulegen, welche Neigung beispielsweise im Wesentlichen dem Winkel zwischen der Hohlraumachse und der Polachse eines vorstehend erläuterten Implantats entspricht. Dabei findet beispielsweise auch ein geeignetes, zum Beispiel mechanisches, Hilfsmittel Verwendung, um den Mittelpunkt M des Femurkopfes zu lokalisieren, damit das Zentrum der Drehung zwischen Halsachse und Implantationsachse dorthin gelegt werden kann. Anschließend wird ein zweiter Bohrdraht 59 in den Femurkopf 49 entlang der festgelegten Implantationsachse A eingesetzt. Danach werden das Ausrichtinstrument 55 und der erste Bohrdraht 57 wieder entfernt. Anschließend wird der zweite Bohrdraht 59 als Führungswerkzeug für ein geeignetes Bearbeitungsinstrument, beispielsweise einen Kegelstumpffräser, benutzt, um den Femurkopf 49 derart zu bearbeiten, dass daraus ein präparierter Stumpf entsteht, der eine Gegenform zu dem Hohlraum eines vorstehend erläuterten Implantats darstellt und rotationssymmetrisch bezüglich der Implantationsachse A ist. Anschließend wird der zweite Bohrdraht 59 wieder entfernt. Danach wird das Implantat, insbesondere ein Implantat der oben beschriebenen Art, derart an den präparierten Stumpf herangeführt und auf diesem aufgesetzt, dass die Hohlraumachse des Implantats und die Implantationsachse zusammenfallen. Dabei kann das Implantat gegen Verkippung der Hohlraumachse gegenüber der Implantatioisachse gesichert werden. Ein nach diesem Verfahren auf einem präparierten Femurkopf 35 implantiertes Implantat ist in Fig. 3 gezeigt. Das Implantat ist dabei derart auf dem präparierten. Femurkopf 35 orientiert, dass die Polachse 15 des Implantats mit der Halsachse N des präparierten Femurkopfes 35 und die Hohlraumachse 19 des Implantats mit der Implantationsachse A, die der Hauptbelastungsrichtung des Femurkopfes entspricht, übereinstimmen oder in der Praxis zumindest in guter Näherung übereinstimmen. In Fig. 3 ist die Implantationsachse A lediglich in einer Frontalebene gegenüber der Femurhalsachse N geneigt. Grundsätzlich kann die Implantationsachse A aber auch zusätzlich in einer Sagittalebene gegenüber der Femurhalsachse N geneigt sein; dies wird als Antetorsion bezeichnet.

Im Lichte der hier gemachten Ausführungen eröffnen sich dem Fachmann weitere Ausführungsformen der in den Ansprüchen gekennzeichneten Erfindung, welche hier nicht abschließend dargestellt werden können.

### Bezugszeichenliste

- 11: Kugelabschnitt
- 13, 13': ebene Basisfläche
- 15: Polachse
- 17: Hohlraum
- 19: Hohlraumachse
- 21: Eintrittsebene
- 23: Stirnfläche
- 25: Mantelfläche
- 27: Verankerungsstift
- 29: Verankerungsfinne
- 31: Randseite einer Verankerungsfinne
- 33: Flachseite einer Verankerungsfinne
- 35: präparierter Stumpf
- 37: Rand der Kappenöffnung
- 39, 39': sphärischer Oberflächenbereich
- 41: Schaft
- 43: Kopfteil
- 45: Prägekante
- 47: Femur
- 49: Femurkopf
- 51: Femurhals
- 53: großer Trochanter
- 55: Ausrichtinstrument
- 57: erster Bohrdraht
- 59: zweiter Bohrdraht
- 171: Erhebungen
- A: Implantationsachse
- M: Mittelpunkt des Kugelabschnitts
- N: Femurhalsachse
- α: Winkel zwischen Pol- und Hohlraumachse
- 2δ: Konusöffnungswinkel

## Patentansprüche

1. Kappenförmig ausgeformtes Implantat zur Implantation auf einem präparierten Stumpf eines Femurkopfes, wobei das Implantat die Form eines Kugelabschnitts (11) aufweist, mit einer ebenen Basisfläche (13) und einer senkrecht zu der Basisfläche (13) angeordneten Polachse (15), in welchem Kugelabschnitt (11) ausgehend von der Basisfläche (13) ein Hohlraum (17) angeordnet ist, welcher Hohlraum (17) rotationssymmetrisch ist und eine Hohlraumachse (19) aufweist, welche Hohlraumachse (19) unter einem von Null verschiedenen Winkel (α) zur Polachse (15) angeordnet ist, wobei die Pol- und Hohlraumachse (15, 19) miteinander einen Winkel (α) einschließen, welcher im Bereich von 15° bis 50°, insbesondere 15° bis 35°, liegt, wobei der Hohlraum (17) eine Innenfläche (25) aufweist, welche eine Durchdringung mit der Oberfläche des Kugelabschnitts (11) aufweist, und wobei wenigstens ein Umfangssegment der Innenfläche (25) eine Durchdringung mit einem sphärischen Oberflächenbereich (39) des Kugelabschnitts (11) aufweist.

2. Implantat nach Anspruch 1,
**dadurch gekennzeichnet,**
**dass** die Hohlraumachse (19) die Polachse (15) schneidet.

3. Implantat nach Anspruch 1 oder 2,
**dadurch gekennzeichnet,**
**dass** der Kugelabschnitt (11) einen Kugelmittelpunkt (M) aufweist, wobei die Hohlraumachse (19) die Polachse (15) im Kugelmittelpunkt (M) schneidet.

4. Implantat nach zumindest einem der vorstehenden Ansprüchen,
**dadurch gekennzeichnet,**
**dass** der Kugelabschnitt (11) einen Kugeldurchmesser aufweist, welcher im Bereich von 38mm bis 60mm liegt.

5. Implantat nach zumindest einem der vorstehenden Ansprüche,
**dadurch gekennzeichnet,**
**dass** der Kugelabschnitt (11) einen Kugeldurchmesser und in Richtung der Polachse (15) eine von der Basisfläche (13) bis zum Pol messende Höhe aufweist, welche Höhe 60% bis 80% des Kugeldurchmessers beträgt.

6. Implantat nach einem der vorhergehenden Ansprüche
**dadurch gekennzeichnet,**
**dass** ein erstes Umfangssegment der Innenfläche (25) den sphärischen Oberflächenbereich (39) des Kugelabschnitts (11) durchdringt und ein zweites Umfangssegment der Innenfläche (25) die Basisfläche (13) durchdringt.

7. Implantat nach zumindest einem der vorstehenden Ansprüche,
**dadurch gekennzeichnet,**
**dass** der Hohlraum die Form eines Kegelstumpfes (17) aufweist, welcher sich entlang der Hohlraumachse (19) in einer Richtung verjüngt, die ins Innere des Implantats hineinweist.

8. Implantat nach Ansprüch 7,
**dadurch gekennzeichnet,**
**dass** der Kegelstumpf (17) einen Konusöffnungswinkel (2δ) aufweist, welcher im Bereich von 10° bis 65°, insbesondere 16° bis 50°, liegt.

9. Implantat nach zumindest einem der vorstehenden Ansprüche,
**dadurch gekennzeichnet,**
**dass** der Hohlraum (17) von einer Innenfläche (23, 25) begrenzt ist, welche Verankerungsmittel (27, 29) zur Verankerung des Implantats auf dem Stumpf aufweist.

10. Implantat nach Anspruch 9,
**dadurch gekennzeichnet,**
**dass** die Verankerungsmittel (27, 29) symmetrisch um die Hohlraumachse (19) verteilt angeordnet sind.

11. Implantat nach Anspruch 9 oder 10,
**dadurch gekennzeichnet,**
**dass** die Verankerungsmittel (27, 29) in den Hohlraum (17) vorstehen.

12. Implantat nach zumindest einem der Ansprüche 9 bis 11,
**dadurch gekennzeichnet,**
**dass** die Verankerungsmittel am Umfang des Hohlraums (17) angeordnete widerhakenartige Strukturen umfassen, welche von einer Öffnung des Hohlraums (17) wegweisend ins Innere des Implantats hinein gebogen sind.

13. Implantat nach zumindest einem der Ansprüche 9 bis 12,
**dadurch gekennzeichnet,**
**dass** die Verankerungsmittel (27, 29) mehrere Verankerungsrippen, -finnen (29) und/oder parallel zur Hohlraumachse (19) orientierte Verankerungsstifte (27) umfassen.

## Claims

1. An implant made in cap shape for implantation on a prepared stump of a femoral head, wherein the implant has the shape of a spherical section (11), comprising a planar base surface (13) and a polar axis (15) arranged perpendicular to the base surface (13), in which spherical section (11) a hollow space (17) is arranged, starting from the base surface (13), said hollow space (17) being rotationally symmetrical and having a hollow space axis (19), said hollow space axis (19) being arranged at an angle (α) different than zero to the polar axis (15); wherein the polar axis and hollow space axis (15, 19) include an angle (α) with one another which lies in the range from 15° to 50°, in particular from 15° to 35°; wherein the hollow space (17) has an inner surface (25) which has a penetration with the surface of the spherical section (11); and wherein at least one peripheral segment of the inner surface (25) has a penetration with a spherical surface region (39) of the spherical section (11).

2. An implant in accordance with claim 1,
**characterized in that**
the hollow space axis (19) intersects the polar axis (15).

3. An implant in accordance with claim 1 or claim 2,
**characterized in that**
the spherical section (11) has a spherical center (M), with the hollow space axis (19) intersecting the polar axis (15) at the spherical center (M).

4. An implant in accordance with at least one of the preceding claims,
**characterized in that**
the spherical section (11) has a spherical diameter lying in the range from 38 mm to 60 mm.

5. An implant in accordance with at least one of the preceding claims,
**characterized in that**
the spherical section (11) has a spherical diameter and a height measured from the base surface (13) up to the pole in the direction of the polar axis (15), said height amounting to 60% to 80% of the spherical diameter.

6. An implant in accordance with any one of the preceding claims,
**characterized in that**
a first peripheral segment of the inner surface (25) penetrates the spherical surface region (39) of the spherical section (11) and a second peripheral segment of the inner surface (25) penetrates the base surface (13).

7. An implant in accordance with at least one of the preceding claims,
**characterized in that**
the hollow space has the shape of a conical stump (17) which tapers along the hollow space axis (19) in a direction facing the interior of the implant.

8. An implant in accordance with claim 7, **characterized in that** the conical stump (17) has a conical opening angle (2δ) lying in the region from 10° to 65°, in particular from 16° to 50°.

9. An implant in accordance with at least one of the preceding claims,
**characterized in that**
the hollow space (17) is bounded by an inner space (23, 25) having anchorage means (27, 29) for the anchorage of the implant on the stump.

10. An implant in accordance with claim 9, **characterized in that** the anchorage means (27, 29) are arranged symmetrically distributed around the hollow space axis (19).

11. An implant in accordance with claim 9 or claim 10, **characterized in that**
the anchorage means (27, 29) project into the hollow space (17).

12. An implant in accordance with at least one of the claims 9 to 11,
**characterized in that**
the anchorage means comprise barb-like structures arranged at the periphery of the hollow space (17), said barb-like structures being bent facing away from an opening of the hollow space (17) inwardly into the interior of the implant.

13. An implant in accordance with at least one of the claims 9 to 12,
**characterized in that**
the anchorage means (27, 29) comprise a plurality of anchorage ribs, anchorage fins (29) and/or anchorage pins (27) oriented parallel to the hollow space axis (19).

## Revendications

1. Implant conformé à la manière d'un capuchon pour l'implantation sur un moignon préparé d'une tête de fémur, ledit implant présentant la forme d'une calotte sphérique (11) avec une surface de base (13) plane et un axe polaire (15) agencé perpendiculairement sur la surface de base (13), avec une cavité (17) ménagée dans cette calotte sphérique (11) en partant de la surface de base (13), ladite cavité (17) présentant une symétrie de révolution et un axe de cavité (19), celui-ci étant agencé par rapport à l'axe polaire (15) sous un angle (α) différent de zéro, tels que l'axe polaire et l'axe de cavité (15, 19) définissent l'un avec l'autre un angle (α) situé dans la plage de 15° à 50°, en particulier de 15° à 35°, la cavité (17) comportant une surface intérieure (25) qui présente une traversée avec la surface de la calotte sphérique (11), et au moins un segment périphérique de la surface intérieure (25) présente une traversée avec une zone de surface sphérique (39) de la calotte sphérique (11).

2. Implant selon la revendication 1,
**caractérisé en ce que** l'axe de cavité (19) recoupe l'axe polaire (15).

3. Implant selon la revendication 1 ou 2,
**caractérisé en ce que** la calotte sphérique (11) présente un centre de sphère (M), tel que l'axe de cavité (19) recoupe l'axe polaire (15) au centre de sphère (M).

4. Implant selon l'une au moins des revendications précédentes,
**caractérisé en ce que** la calotte sphérique (11) présente un diamètre qui est dans la plage de 38 mm à 60 mm.

5. Implant selon l'une au moins des revendications précédentes,
**caractérisé en ce que** la calotte sphérique (11) présente un diamètre et, en direction de l'axe polaire (15), une hauteur, mesurée de la surface de base (13) jusqu'au pôle, qui s'élève à 60 % à 80 % du diamètre.

6. Implant selon l'une des revendications précédentes,
**caractérisé en ce qu'**un premier segment périphérique de la surface intérieure (25) traverse la zone de surface sphérique (39) de la calotte sphérique (11), et un second segment périphérique de la surface intérieure (25) traverse la surface de base (13).

7. Implant selon l'une au moins des revendications précédentes,
**caractérisé en ce que** la cavité a la forme d'un tronc de cône (17), lequel va en se rétrécissant le long de l'axe de cavité (19) dans une direction qui va vers l'intérieur de l'implant.

8. Implant selon la revendication 7,
**caractérisé en ce que** le tronc de cône (17) présente un angle d'ouverture conique (26) qui est dans la plage de 10° à 65°, en particulier 16° à 50°.

9. Implant selon l'une au moins des revendications précédentes,
**caractérisé en ce que** la cavité (17) est délimitée par une surface intérieure (23, 25), laquelle comporte des moyens d'ancrage (27, 29) pour l'ancrage de l'implant sur le moignon.

10. Implant selon la revendication 9,
**caractérisé en ce que** les moyens d'ancrage (27, 29) sont agencés de manière répartie symétriquement autour de l'axe de cavité (19).

11. Implant selon la revendication 9 ou 10,
**caractérisé en ce que** les moyens d'ancrage (27, 29) dépassent dans la cavité (17).

12. Implant selon l'une au moins des revendications 9 à 11,
**caractérisé en ce que** les moyens d'ancrage comprennent des structures semblables à des ardillons agencés à la périphérie de la cavité (17), lesquels sont recourbés en éloignement d'une ouverture de la cavité (17) en entrant dans l'intérieur de l'implant.

13. Implant selon l'une au moins des revendications 9 à 12,
**caractérisé en ce que** les moyens d'ancrage (27, 29) comprennent plusieurs nervures d'ancrage, ailettes d'ancrage (29), et/ou tiges d'ancrage (27) orientées parallèlement à l'axe de cavité (19).
